# EUROPEAN PATENT APPLICATION

(11) **EP 4 364 734 A1**
(43) Date of publication of application: **08.05.2024**
(21) Application number: 22833577.4
(22) Date of filing: 28.06.2022
(51) Int. Cl.: A61K 31/4166, A61P 13/12, A23K 20/137

(54) **VETERINARY PHARMACEUTICAL COMPOSITION COMPRISING 5-HYDROXY-1-METHYLIMIDAZOLIDINE-2,4-DIONE COMPOUND AS ACTIVE INGREDIENT**

(30) Priority: 02.07.2021 KR 20210087121; 15.06.2022 KR 20220072906
(71) Applicant: Kyongbo Pharm. Co., Ltd., Asan-si, Chungcheongnam-do 31501 (KR); Ndic Co., Ltd., Hwaseong-si, Gyeonggi-do 18469 (KR)
(72) Inventor: HAN, Jeong Seok, Anyang-si Gyeonggi-do 13922 (KR); PARK, Yong Kyu, Suwon-si Gyeonggi-do 16324 (KR); BANG, Sung Hun, Cheongju-si Chungcheongbuk-do 28124 (KR); KIM, Jae Hyun, Yongin-si Gyeonggi-do 17065 (KR); JANG, Ha Lim, Suwon-si Gyeonggi-do 16484 (KR); KIM, Ye Rin, Yongin-si Gyeonggi-do 16892 (KR); LEE, Su Kyoung, Cheonan-si Chungcheongnam-do 31171 (KR); LIM, Taek Joo, Hwaseong-si Gyeonggi-do 18447 (KR)
(74) Representative: Murgitroyd & Company
(86) International application number: PCT/KR2022/009222
(87) International publication number: WO 2023/277521

(57) **Abstract**

Disclosed are a pharmaceutical composition for animals containing 5-hydroxy-1-methylimidazolidine-2,4-dione as an active ingredient, more specifically, a composition for preventing or treating kidney disease in animals containing 5-hydroxy-1-methylimidazolidine-2,4-dione or a pharmaceutically acceptable salt thereof as an active ingredient, a method of treating kidney disease in animals using the compound, and a functional feed for preventing or ameliorating kidney disease in animals containing the compound as an active ingredient. The 5-hydroxy-1-methylimidazolidine-2,4-dione can effectively ameliorate and treat symptoms of kidney disease when administered to companion animals such as dogs or cats with kidney disease. Therefore, there are provided a novel agent and method for treating kidney disease of animals that can effectively treat kidney disease, which is the cause of high mortality in companion animals.

## Description

### [Technical Field]

The present invention relates to a pharmaceutical composition for animals containing 5-hydroxy-methylimidazolidine-2,4-dione as an active ingredient. More specifically, the present invention relates to a pharmaceutical composition for preventing or treating kidney disease in animals containing 5-hydroxy-methylimidazolidine-2,4-dione as an active ingredient.

### [Background Art]

As the number of populations raising companion animals rapidly increases, the animal medical market continues to grow, pharmaceutical and biotechnology companies in Korea and other nations are actively conducting research to develop products such as medicine, feed, or nutritional supplements for companion animals.

The global veterinary drug market exceeded 37 trillion Won as of 2018, the market has grown by an annual average of 5% or more over the past five years, and Transparency Market Research (TMR), which is an American market research company, expects that the total veterinary pharmaceutical market will grow to $50.2 billion (approximately 55 trillion Won) in 2024.

Meanwhile, the kidneys are essential organs that are responsible for producing and regulating blood in animals. Kidney malfunction may cause serious situations. Damaged kidneys have difficulty returning to a normal state. In particular, the kidneys filter out waste and salt from the body, re-absorb essential ingredients and produce urine to regulate body moisture. However, malfunction of the kidneys may accumulate waste in the body, causing various symptoms such as uremia and hyperkalemia.

Kidney damage in companion animals is not easily visible and thus it is difficult to recognize the symptoms thereof. In many cases, pets die when symptoms have already appeared. This is why kidney disease is one of the top three causes of death in companion animals.

In addition, it is difficult for companion animals such as dogs and cats, unlike humans, to actively consume water to prevent kidney disease, and most food for companion animals is dry-type, making it impossible for them to consume enough water through meals.

Therefore, there is a need to develop a therapeutic agent for kidney disease of companion animals that can effectively prevent or treat kidney disease, which is a leading cause of death in companion animals.

### [Disclosure]

### [Technical Problem]

Accordingly, while researching a novel therapeutic agent and method for effectively treating kidney disease of companion animals, the present inventors developed an optimal treatment method based on the finding that 5-hydroxy-1-methylimidazolidine-2,4-dione can effectively ameliorate and treat kidney disease of companion animals, thereby completing the present invention.

Therefore, it is one object of the present invention to provide a composition for preventing or treating kidney disease in animals containing 5-hydroxy-1-methylimidazolidine-2,4-dione or a pharmaceutically acceptable salt thereof as an active ingredient.

It is another object of the present invention to provide a method of treating kidney disease in animals using 5-hydroxy-1-methylimidazolidine-2,4-dione or a pharmaceutically acceptable salt thereof.

It is another object of the present invention to provide a functional feed for preventing or ameliorating kidney disease in animals containing 5-hydroxy-1-methylimidazolidine-2,4-dione or a pharmaceutically acceptable salt thereof as an active ingredient.

### [Technical Solution]

In accordance with one aspect of the present invention, the above and other objects can be accomplished by the provision of a composition for preventing or treating kidney disease in animals containing 5-hydroxy-1-methylimidazolidine-2,4-dione or a pharmaceutically acceptable salt thereof as an active ingredient.

In an embodiment of the present invention, the kidney disease may be selected from the group consisting of nephritis, pyelonephritis, nephrotic syndrome, kidney cancer, acute pyelonephritis, chronic pyelonephritis, renal tuberculosis, urinary tract infection, urinary stone (calculi), ureter stone, acute renal failure, chronic renal failure, diabetic nephropathy, chronic glomerulonephritis, acute progressive nephritis, nephrosis syndrome, microglial glomerulosclerosis, membranous nephropathy, and membranous proliferative glomerulonephritis occurring in animals.

In an embodiment of the present invention, the animal may be a dog or a cat.

In an embodiment of the present invention, the 5-hydroxy-1-methylimidazolidine-2,4-dione may be orally administered at a daily dose of 25 to 200 mg per kg of weight to an animal with chronic kidney disease having a serum creatinine (sCr) level of 1.4 mg/dL or higher.

In an embodiment of the present invention, the dose may be 25 mg to 100 mg per kg of weight.

In accordance with another aspect of the present invention, provided is a method of treating kidney disease in animals including administering a composition containing 5-hydroxy-1-methylimidazolidine-2,4-dione or a pharmaceutically acceptable salt thereof to an animal with kidney disease.

In an embodiment of the present invention, the animal with kidney disease may be an animal with chronic kidney disease having a serum creatinine (sCr) level of 1.4 mg/dL or higher.

In an embodiment of the present invention, the kidney disease may be selected from the group consisting of nephritis, pyelonephritis, nephrotic syndrome, kidney cancer, acute pyelonephritis, chronic pyelonephritis, renal tuberculosis, urinary tract infection, urinary stone (calculi), ureter stone, acute renal failure, chronic renal failure, diabetic nephropathy, chronic glomerulonephritis, acute progressive nephritis, nephrosis syndrome, microglial glomerulosclerosis, membranous nephropathy, and membranous proliferative glomerulonephritis occurring in animals.

In an embodiment of the present invention, the animal may be a dog or a cat.

In an embodiment of the present invention, the administration may be oral administration.

In accordance with another aspect of the present invention, provided is a functional feed for preventing or ameliorating kidney disease in animals containing 5-hydroxy-1-methylimidazolidine-2,4-dione or a pharmaceutically acceptable salt thereof as an active ingredient.

### [Advantageous effects]

The 5-hydroxy-1-methylimidazolidine-2,4-dione according to the present invention can effectively ameliorate and treat symptoms of kidney disease when administered to companion animals such as dogs or cats with kidney disease. Therefore, the present invention has the effect of providing a novel agent and method for treating kidney disease of animals that can effectively treat kidney disease, which is the cause of high mortality in companion animals.

### [Description of Drawings]

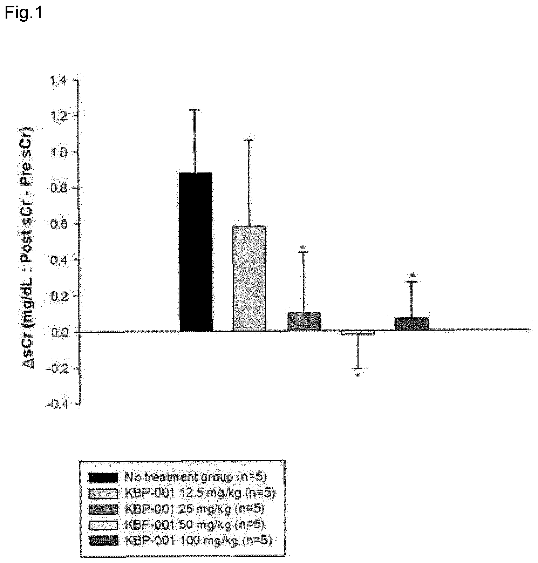

FIG. 1 illustrates changes in blood creatinine before and after administration of 5-hydroxy-1-methylimidazolidine-2,4-dione to dogs with chronic renal failure.
FIG. 2 is a calibration curve of 5-hydroxy-1-methylimidazolidine-2,4-dione (concentration range of 50 to 100,000 ng/nL) in the plasma of dogs with chronic renal failure.
FIG. 3 shows an average concentration of 5-hydroxy-1-methylimidazolidine-2,4-dione in the plasma over time after oral administration to dogs with chronic renal failure.

### [Best Mode]

The present invention is directed to a composition for effectively preventing or treating kidney disease of animals, more specifically, a composition for preventing or treating kidney disease of animals containing 5-hydroxy-1-methylimidazolidine-2,4-dione or a pharmaceutically acceptable salt thereof as an active ingredient.

The number of populations living with companion animals is increasing worldwide, including in Korea, and the lifespan of companion animals is also increasing due to the development of functional feeds.

Meanwhile, as the lifespan of companion animals increases, the occurrence of diseases in companion animals is also increasing. In particular, kidney disease is known to be a major cause of death in companion animals. However, veterinary medicines that can effectively treat kidney disease in companion animals have not been developed.

The kidney is an organ that produces and regulates blood in animals and performs a variety of functions, including waste excretion, blood pressure regulation, urine concentration, metabolite excretion, and secretion of red blood cell-producing hormones. When these kidney functions are lost or impaired, kidney disease occurs. Representative kidney diseases include acute renal failure and chronic renal failure. Acute renal failure may result in kidney failure in a short period of time due to kidney damage caused by toxic substances or blockage of the ureters or urethra, and in severe cases, may cause death within a few days. Chronic renal failure refers to a disease in which kidneys are gradually damaged and lose functions over several months to years due to various causes such as congenital anomalies, kidney cysts, kidney stones, immune abnormalities, infections, and tumors.

In particular, chronic renal failure is known to be a major cause of death in older dogs or cats. When kidney function declines significantly due to aging, toxin levels rise rapidly and various systemic symptoms appear. Typical symptoms include vomiting, loss of appetite, increased skin keratin, hair dullness, increased water consumption and urine output, and the like.

However, by the time the owner discovers these symptoms in companion animals, there is a high probability that more than 75% of the kidneys have already been damaged.

In addition, acute renal failure may cause quick death of companion animals due to rapid worsening of symptoms.

Meanwhile, there are currently neither therapeutic agents nor methods that can effectively prevent or treat kidney disease in companion animals.

Accordingly, the present invention has identified that 5-hydroxy-1-methylimidazolidine-2,4-dione can be useful as a medicine for kidney disease in animals that can effectively prevent, ameliorate, and treat kidney disease in animals. The optimal conditions for administering the compound to companion animals were established.

In an embodiment of the present invention, 5-hydroxy-1-methylimidazolidine-2,4-dione is administered at different doses to beagles with chronic renal failure having a serum creatinine (sCr) level of 1.4 mg/dL or more and the result shows the administration of the compound alleviates and relieves symptoms of chronic renal failure.

In addition, it was found that the compound is preferably administered at a dose of 25 to 200 mg per kg of weight of the beagle per day, and can most effectively prevent, ameliorate, and treat chronic renal failure when administered at a dose of 50 mg.

Meanwhile, to date, there has been no case of identifying administration of 5-hydroxy-1-methylimidazolidine-2,4-dione to companion animals as having a therapeutic effect on kidney disease. However, the present invention is characterized by establishing the administration conditions of 5-hydroxy-1-methylimidazolidine-2,4-dione that can quickly and effectively treat kidney disease in companion animals.

In particular, determining the dose of a specific drug to induce pharmacological effects in different subjects can be identified only by actual experimentation.

The present inventors conducted 1-compartment model analysis on 5-hydroxy-1-methylimidazolidine-2,4-dione to predict the movement of the drug *in vivo* in beagles. As a result, the predicted effective dose was approximately 31.06 mg/kg.

Based on these results, the present inventors analyzed the level of creatinine in the blood as an indicator showing the effect of oral administration of different concentrations of 5-hydroxy-1-methylimidazolidine-2,4-dione to beagles with chronic renal failure on amelioration and treatment of symptoms, and the results showed that administration of the compound at a dose of 50 mg/kg was more effective compared to administration thereof at other doses.

In consideration of these aspects, the appropriate dose of a specific drug to obtain the optimal pharmacological effect therefrom can be determined through the results of actual experiments, rather than prediction methods known in the art.

In addition, different subjects differ in drug kinetics, such as surface area and bioavailability. Therefore, optimization of the efficacy of a drug for a specific subject can be determined only from the results of actual experiments targeting the subject.

In this regard, the present invention established for the first time the optimal dosage for optimizing the therapeutic efficacy of 5-hydroxy-1-methylimidazolidine-2,4-dione on dogs with kidney disease.

Therefore, the present invention is directed to a composition for preventing or treating kidney disease in animals containing 5-hydroxy-1-methylimidazolidine-2,4-dione or a pharmaceutically acceptable salt thereof as an active ingredient.

The kidney disease may be selected from the group consisting of nephritis, pyelonephritis, nephrotic syndrome, kidney cancer, acute pyelonephritis, chronic pyelonephritis, renal tuberculosis, urinary tract infection, urinary stone (calculi), ureter stone, acute renal failure, chronic renal failure, diabetic nephropathy, chronic glomerulonephritis, acute progressive nephritis, nephrosis syndrome, microglial glomerulosclerosis, membranous nephropathy, and membranous proliferative glomerulonephritis occurring in animals.

In addition, the composition of the present invention aims at preventing or treating kidney disease occurring in animals, preferably companion animals, and the companion animal may be a dog or a cat, and more preferably a dog, but is not limited thereto.

The animal with kidney disease in need of treatment using the composition of the present invention may be an animal with chronic renal failure stages 2 to 4 having a serum creatinine (sCr) level of 1.4 mg/dL or more.

Stages of chronic renal failure in companion dogs are divided into stages 1 to 4. In the case where there is no azotemia and the level of creatinine is less than 1.4 mg/dL, or the level of SDMA (ug/dL) is less than 18, or UPC ratio is less than 0.2, or the systolic blood pressure (mmHg) is less than 140, it is diagnosed as stage 1.

In addition, in the case where there is mild azotemia and the level of creatinine is 1.4 to 2.8 mg/dL, or the level of SDMA (ug/dL) is 18 to 35, or a UPC ratio is 0.2 to 0.5, or the systolic blood pressure (mmHg) is within a pre-hypertension range of 140 to 159, it is diagnosed as stage 2.

In addition, in the case where there is moderate azotemia and the level of creatinine is 2.9 to 5.0 mg/dL, or the level of SDMA (ug/dL) is 36 to 54, or a UPC ratio is 0.2 to 0.5, or the systolic blood pressure (mmHg) is within a hypertension range of 160 to 179, it is diagnosed as stage 3.

In addition, in the case where there is severe azotemia and the level of creatinine is higher than 5.0 mg/dL, or the level of SDMA (ug/dL) is higher than 54, or a UPC ratio is higher than 0.5, or the systolic blood pressure (mmHg) is within a severe hypertension range of 180 or more, it is diagnosed as stage 4.

In an embodiment of the present invention, 5-hydroxy-1-methylimidazolidine-2,4-dione may be orally administered at a daily dose of 25 to 200 mg per kg of weight, preferably, at a daily dose of 25 to 100 mg per kg of weight, most preferably, at a daily dose of 50 mg per kg of weight, to an animal with chronic kidney disease having a serum creatinine (sCr) level of 1.4 mg/dL or higher.

In the present invention, the composition for preventing or treating kidney disease of animals may contain a pharmaceutically acceptable acid or base addition salt. Examples of the acid addition salt include, but are not limited to, inorganic acid salts derived from hydrochloric acid, nitric acid, phosphoric acid, bromic acid, iodic acid, perchloric acid, tartaric acid and sulfuric acid, organic acid salts derived from acetic acid, trifluoroacetic acid, citric acid, maleic acid, succinic acid, oxalic acid, benzoic acid, tartaric acid, fumaric acid, mandelic acid, propionic acid, citric acid, lactic acid, glycolic acid, gluconic acid, galacturonic acid, glutamic acid, glutaric acid, glucuronic acid, aspartic acid, ascorbic acid, carbonic acid, vanillic acid, and hydroiodic acid, and sulfonic acid salts derived from methanesulfonic acid, ethanesulfonic acid, benzenesulfonic acid, p-toluenesulfonic acid, naphthalenesulfonic acid, and the like, and examples of the base addition salt include, but are not limited to, ammonium salts, alkali and alkaline earth metal salts, sodium, potassium, magnesium, calcium salts, and the like. In addition, the types of salts that may be used in the present invention are not limited by the salts mentioned above.

In addition, the composition of the present invention may further contain a pharmaceutically acceptable additive, such as a diluent, binder, disintegrant, lubricant, pH adjuster, antioxidant, or solubilizer, which is pharmaceutically acceptable without impairing the effects of the present invention.

The diluent may be sugar, starch, microcrystalline cellulose, lactose (lactose hydrate), glucose, di-mannitol, alginate, an alkaline earth metal salt, clay, polyethylene glycol, anhydrous calcium hydrogen phosphate, or a mixture thereof, and the binder may be starch, microcrystalline cellulose, highly dispersed silica, mannitol, D-mannitol, sucrose, lactose hydrate, polyethylene glycol, polyvinylpyrrolidone (povidone), a polyvinylpyrrolidone copolymer (copovidone), hypromellose, hydroxypropyl cellulose, natural gum, synthetic gum, copovidone, gelatin, or a mixture thereof.

The disintegrant may be starch or modified starch such as sodium starch glycolate, corn starch, tapioca starch, potato starch, or pregelatinized starch, clay such as bentonite, montmorillonite, or Veegum, cellulose such as microcrystalline cellulose, hydroxypropyl cellulose, or carboxymethyl cellulose, algin such as sodium alginate or alginic acid, cross-linked cellulose such as croscarmellose sodium, gum such as guar gum and xanthan gum, a cross-linked polymer such as cross-linked polyvinylpyrrolidone (crospovidone), an effervescent agent such as sodium bicarbonate, citric acid, or a mixture thereof.

The lubricant may be talc, stearic acid, magnesium stearate, calcium stearate, sodium lauryl sulfate, hydrogenated vegetable oil, sodium benzoate, sodium stearyl fumarate, glyceryl behenate, glyceryl monooleate, glyceryl monostearate, glyceryl palmitostearate, colloidal silicon dioxide, a mixture thereof or the like.

The pH adjuster may be an acidifier such as acetic acid, adipic acid, ascorbic acid, sodium ascorbate, sodium etherate, malic acid, succinic acid, tartaric acid, fumaric acid, or citric acid, or an alkalinizing agent such as precipitated calcium carbonate, aqueous ammonia, meglumine, sodium carbonate, magnesium oxide, magnesium carbonate, sodium citrate, or tribasic calcium phosphate.

The antioxidant may be dibutyl hydroxytoluene, butylated hydroxyanisole, tocopherol acetate, tocopherol, propyl gallate, sodium bisulfite, sodium pyrosulfite, or the like. The solubilizer in the pre-release compartment of the present invention may be sodium lauryl sulfate, polyoxyethylene sorbitan fatty acid ester such as polysorbate, docusate sodium, poloxamer, or the like.

In addition, the pharmaceutical composition according to the present invention may be prepared as an oral formulation such as a powder, granule, tablet, capsule, suspension, emulsion, syrup or aerosol, an external preparation, suppository, or a sterile injection solution in accordance with a conventional method. Preferred is an oral formulation.

Furthermore, the present invention is directed to a method of treating kidney disease of animals including administering a composition containing 5-hydroxy-1-methylimidazolidine-2,4-dione or a pharmaceutically acceptable salt thereof to an animal with kidney disease.

As used herein, the term "administration" refers to introducing the composition for preventing or treating kidney disease of animals according to the present invention into a companion animal using any appropriate method, and any common route may be used as the route of administration of the composition of the present invention as long as the composition can reach the target tissue. The route of administration may be performed by oral, intraperitoneal, intravenous, intramuscular, subcutaneous, endothelial, intranasal, intrapulmonary, intrarectal, intravenous, intraperitoneal, or intrathecal administration, preferably oral administration.

The composition according to the present invention may be administered once a day, or two or more times a day at regular time intervals.

The animal with kidney disease may be an animal with chronic kidney disease having a serum creatinine (sCr) level of 1.4 mg/dL or higher and may be a dog or cat.

Furthermore, the present invention is directed to a functional feed composition for preventing or ameliorating kidney disease in animals containing 5-hydroxy-1-methylimidazolidine-2,4-dione or a pharmaceutically acceptable salt thereof as an active ingredient.

In this case, the 5-hydroxy-1-methylimidazolidine-2,4-dione or a salt thereof may be present in an amount of 0.000001 to 50% by weight based on the total weight of the composition and the administration of the composition can effectively prevent or ameliorate kidney disease in animals.

In addition, the functional feed composition of the present invention may be added to general food for companion animals so that it can be ingested by companion animals, and the companion animals may be dogs or cats, but are not limited thereto.

### [Mode for Invention]

The present invention will be described in more detail with reference to the following examples. These examples are provided only for better understanding of the present invention and should not contused as limiting the scope of the present invention.

### <Example 1>

### Therapeutic effect of 5-hydroxy-1-methylimidazolidine-2,4-dione on dogs with chronic renal failure and establishment of optimal dosage thereof

An experiment was conducted to determine the therapeutic effect and optimal dosage of 5-hydroxy-1-methylimidazolidine-2,4-dione in animal kidney disease. Dogs (5 dogs per group) diagnosed with chronic renal failure and with a blood creatinine concentration of 1.4 mg/dL or higher were treated with 5-hydroxy-methylimidazolidine-2,4-dione at four doses of 0, 12.5, 25, 50, and 100 mg/kg for 2 months, and the change in blood creatinine concentration (ΔsCr) before and after administration was determined.

**[Table 1]**

| Animal experimental group | | |
|---|---|---|
| Experimental group | Dose | Number of subjects in each group (number of animals) |
| Untreated group (Control group) | 0 | 5 |
| KBP-001 low-dose treated group | 12.5 mg/kg | 5 |
| KBP-001 medium-dose treated group | 25 mg/kg | 5 |
| KBP-001 high-dose treated group | 50 mg/kg | 5 |
| KBP-001 highest-dose treated group | 100 mg/kg | 5 |

**[Table 2]**

| Results of analysis of creatinine level as function of drug dose | | | |
|---|---|---|---|
| Experimental group | Creatinine level in blood before administration | Creatinine level in blood after administration | ΔsCr |
| Untreated group (Control group) | 2.80 **±** 1.09 mg/dL | 3.68 **±** 1.44 mg/dL | 0.88 **±** 0.33 mg/dL |
| KBP-001 low-dose treated group | 2.84 **±** 0.81 mg/dL | 3.42 **±** 0.43 mg/dL | 0.58 **±** 0.49 mg/dL |
| KBP-001 medium-dose treated group | 2.46 **±** 0.80 mg/dL | 2.56 **±** 1.03 mg/dL | 0.10 **±** 0.34 mg/dL |
| KBP-001 high-dose treated group | 3.02 **±** 0.59 mg/dL | 3.00 **±** 0.52 mg/dL | -0.02 **±** 0.22 mg/dL |
| KBP-001 highest-dose treated group | 2.95 **±** 0.78 mg/dL | 3.02 **±** 0.26 mg/dL | 0.07 **±** 0.20 mg/dL |

The result of the analysis showed that the untreated group (control group) exhibited an increase in the blood creatinine level by 0.88 ± 0.33 mg/dL, compared to before administration, and the group administered 12.5 mg/kg of 5-hydroxy-1-methylimidazolidine-2,4-dione exhibited an increase in the blood creatinine level by 0.58 ± 0.49 mg/dL compared to before administration. Meanwhile, the group administered 25 mg/kg of 5-hydroxy-1-methylimidazolidine-2,4-dione, the group administered 50 mg/kg of 5-hydroxy-1-methylimidazolidine-2,4-dione, and the group administered 100 mg/kg of 5-hydroxy-1-methylimidazolidine-2,4-dione exhibited decreases in the blood creatinine level by 0.10 ± 0.34 mg/dL, -0.02 ± 0.22 mg/dL, and 0.07 ± 0.20 mg/dL, respectively, compared to before administration, in other words, these groups inhibited significant increases in blood creatinine level compared to the untreated group. These results supported that the 5-hydroxy-1-methylimidazolidine-2,4-dione according to the present invention was found to be usable as a therapeutic agent for renal failure in animals that is capable of treating chronic renal failure in companion animals, administration of 5-hydroxy-1-methylimidazolidine-2,4-dione at a dose of 25 mg/kg or more is effective for inhibition or amelioration of chronic renal failure in companion animals, and that, and in particular, administration of 5-hydroxy-1-methylimidazolidine-2,4-dione at a dose of 50 mg/kg or more is most effective for inhibition or amelioration of chronic renal failure in companion animals.

### <Example 2>

### Pharmacokinetic analysis of 5-hydroxy-1-methylimidazolidine-2,4-diones in beagles

The present inventors conducted a pharmacokinetic analysis of the 5-hydroxy-1-methylimidazolidine-2,4-dione in beagles. Pharmacokinetics is the analysis of the pharmacological effects of a drug on the living body, including the effects of a specific administered drug on the living body, mechanisms of absorption and distribution, chemical changes in substances in the body, and effects of drug metabolites and excretion pathways drug metabolism. The present inventors analyzed the actual pharmacokinetics of 5-hydroxy-1-methylimidazolidine-2,4-dione in beagles.

For this purpose, 5-hydroxy-1-methylimidazolidine-2,4-dione was weighed, based on the fasting weight measured on the day of administration, using an electronic scale in accordance with the oral doses of 10, 50, 100, and 200 mg/kg for each experimental group, and was added to a 500 ml glass container, DW (distilled water) was added as an excipient, and the drug was dissolved therein through vortexing and stirring. Then, the beagles used in the subsequent experiments were acclimatized and environmentally adapted for about a week before the test, and then the compound was orally administered to each beagle at the doses listed in the table below. In addition, the beagles used in this experiment were bred and kept at the Endik Institute.

**[Table 3]**

| Experimental group | | | | | |
|---|---|---|---|---|---|
| Group | Dose (mg/kg) | Volume (ml/kg) | Administration route | The number (N) of subjects | Subject numbers |
| G1 | 10 | 5 | Oral administration | 5 | 1-5 |
| G2 | 50 | 5 | Oral administration | 5 | 6-10 |
| G3 | 100 | 5 | Oral administration | 5 | 11-15 |
| G4 | 200 | 5 | Oral administration | 5 | 16-20 |

The compound was administered orally, blood was collected in a volume of 3 ml using a syringe from the jugular vein of the experimental beagle at each time (0 (before administration), 0.25, 0.5, 1, 1.5, 2, 4, 8, 12, and 24 hours), the collected blood was centrifuged at 4,000 rpm/10 minutes at 4°C and dispensed into 500 µl of a set of two separate 1.5 ml tubes, and the remaining plasma was dispensed into another set and immediately stored in a deep freezer at -70°C until analysis. The concentration of 5-hydroxy-1-methylimidazolidine-2,4-dione in the collected plasma was measured using the following method.

First, a standard sample solution for preparing a calibration curve was prepared. 1.2 mg of standard 5-hydroxy-1-methylimidazolidine-2,4-dione was dissolved in 0.12 ml of DMSO to prepare a stock solution having a final concentration of 10 mg/ml. Then, the stock solution was diluted to a final concentration of 500, 1,000, 2,000, 5,000, 10,000, 30,000, 100,000, 300,000, and 1,000,000 ng/ml with 70% acetonitrile as a solvent. Then, for an internal standard sample solution, 5.0 mg of 4-methylumbelliferone was dissolved in acetonitrile to prepare a final stock solution of 5 mg/ml, which was then diluted with acetonitrile to obtain a final concentration of 200 ng/ml.

To prepare a calibration curve using each solution prepared above, standard 5-hydroxy-1-methylimidazolidine-2,4-dione was dissolved in DMSO to prepare a 10 mg/ml standard stock solution and then the standard stock solution was frozen at -20°C, and the prepared standard stock solution was diluted with 70% acetonitrile to prepare a working solution. The working solution was diluted with blank plasma stored frozen below -20°C (working standard solution 10%, blank plasma 90%) to prepare standard plasma to adjust the concentration of analyte in plasma to 50, 100, 200, 500, 1,000, 3,000, 10,000, 30,000, and 100,0000 ng/ml. 20 µl of each standard plasma was placed in a 1.5 ml micro-tube and then 180 µl of internal standard solution (200 ng/ml in ACN) was added thereto. After vortexing for 5 minutes, centrifugation was performed at 4°C and 17,600×g for 5 minutes. 150 µl of the supernatant was transferred to a 300 µl screw vial, 5 µl of the supernatant was injected into the HPLCMS/MS system, and a calibration curve was plotted at the ratio of the peak area of the analyte to the obtained peak area of the internal standard.

Then, the plasma sample collected from the beagle was subjected to the following pretreatment for assay. 180 µl of the internal standard solution was added to 20 µl of the plasma sample to induce protein precipitation. Then, the mixed sample was suspended using a vortex mixer for 5 minutes, centrifuged at 17,600 × g for 5 minutes, and 150 µl of the supernatant was transferred to an analysis container. Then, 5 µl of the supernatant was injected into a HPLC/MS/MS system and analysis was performed. The instruments used for analysis were an HPLC system (Agilent 1100 Series with on-line degasser, binary pump, thermostatted well-plate autosampler and column compartment) and an MS/MS system (SCIEX 4000 QTRAP System).

The concentration of the analyte (5-hydroxy-1-methylimidazolidine-2,4-dione) in the plasma sample was determined from the previously prepared calibration curve by calculating the peak area ratio of the analyte to the peak area of the internal standard from the obtained chromatogram. In addition, PK parameters were calculated using a non-compartment model based on data on changes in plasma concentration of drugs over time (WinNonLin software ver. 5.0.1.).

**[Table 4]**

| Weight analysis | | | |
|---|---|---|---|
| **Group** | **Time point (b)** | **Animal No.** | **BW (kg)** |
| **G1** | **0, 0.25, 0.5, 1, 1.5, 2, 4, 8, 12, 24** | **1** | **11.3** |
| | | **2** | **10.0** |
| | | **3** | **11.2** |
| | | **4** | **10.1** |
| | | **5** | **10.7** |
| **G2** | | **6** | **11.3** |
| | | **7** | **9.1** |
| | | **8** | **9.7** |
| | | **9** | **11.8** |
| | | **10** | **11.6** |
| **G3** | | **11** | **9.6** |
| | | **12** | **10.3** |
| | | **13** | **10.6** |
| | | **14** | **10.4** |
| | | **15** | **12.4** |
| **G4** | | **16** | **10.6** |
| | | **17** | **9.7** |
| | | **18** | **10.8** |
| | | **19** | **11.2** |
| | | **20** | **11.0** |

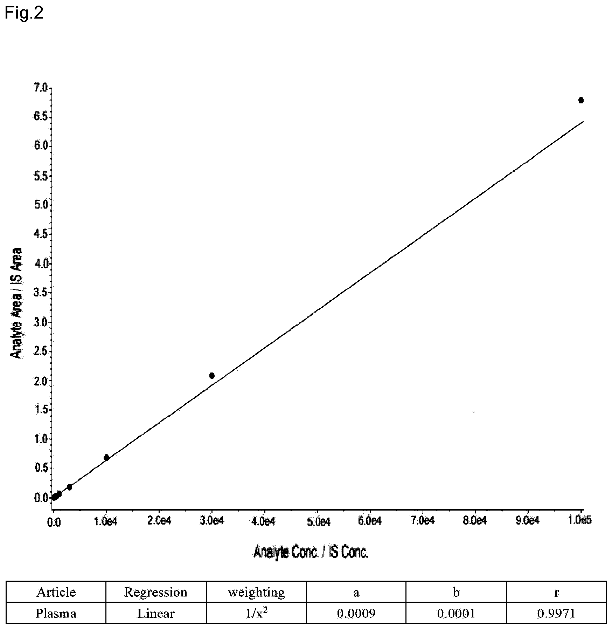

The result of the analysis showed that no adverse reactions were observed until the end of the test after administering the compound to the experimental beagles. The results of weight analysis are shown in Table 4 above. The calibration curve for quantitative analysis of the compound in the plasma of beagles showed that the correlation coefficient (r) of the calibration curve in the quantitative range of 50 ng/ml to 100,000 ng/ml was 0.9972, satisfying the criteria (correlation coefficient of 0.9900 or more) (FIG. 2).

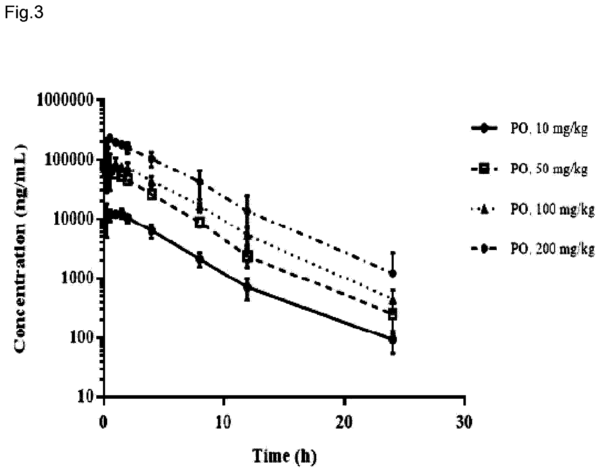

The result of PK profile analysis for the 5-hydroxy-1-methylimidazolidine-2,4-dione according to the present invention showed that, when the compound was orally administered at doses of 10, 50, 100, and 200 mg/kg, the average highest plasma blood concentrations were 15,040.00 ng/ml, 2,004.49 ng/ml, 101,540.00 ng/ml, and 233,400.00 ng/ml at 0.60, 0.35, 0.80, and 0.45 hours, respectively, and gradually decreased over time (FIG. 3 and Table 5).

**[Table 5]**

| Result of analysis of concentration of 5-hydroxy-1-methylimidazolidine-2,4-dione in plasma after oral administration to beagles | | | | | | | | |
|---|---|---|---|---|---|---|---|---|
| **Group (Dose)** | **Time (hr)** | **Concentration (ng/ml)** | | | | | **Mean** | **SD** |
| | | **Animal No.** | | | | | | |
| | | **1** | **2** | **3** | **4** | **5** | | |
| **G1 (10mg/kg)** | **0** | **0.00** | **0.00** | **0.00** | **0.00** | **0.00** | **0.00** | **0.00** |
| | **0.25** | **16300.00** | **12400.00** | **16500.00** | **261.00** | **11900.00** | **11472.20** | **6620.45** |
| | **0.5** | **13500.00** | **12900.00** | **12800.00** | **7100.00** | **12800.00** | **11820.00** | **2654.62** |
| | **1** | **12800.00** | **10200.00** | **11600.00** | **14900.00** | **11200.00** | **12140.00** | **1802.22** |
| | **1.5** | **12800.00** | **11800.00** | **11300.00** | **16700.00** | **10600.00** | **12640.00** | **2406.87** |
| | **2** | **11500.00** | **8640.00** | **9530.00** | **13100.00** | **10000.00** | **10554.00** | **1760.39** |
| | **4** | **6140.00** | **5840.00** | **4560.00** | **9060.00** | **6190.00** | **6358.00** | **1649.07** |
| | **8** | **2610.00** | **1580.00** | **1860.00** | **2850.00** | **1720.00** | **2124.00** | **568.36** |
| | **12** | **983.00** | **424.00** | **633.00** | **1020.00** | **479.00** | **707.80** | **279.15** |
| | **24** | **92.50** | **BQL** | **55.20** | **131.00** | **BQL** | **92,90** | **37.90** |
| **G2 (50mg/kg)** | **0** | **0.00** | **0,00** | **0.00** | **0.00** | **0.00** | **0.00** | **0.00** |
| | **0.25** | **34800.00** | **81400.00** | **45200.00** | **85100.00** | **85900.00** | **66480.00** | **24509.73** |
| | **0.5** | **65100.00** | **54600.00** | **62400.00** | **79100.00** | **79400.00** | **68120.00** | **10867.70** |
| | **1** | **56400.00** | **58100.00** | **55700.00** | **62700.00** | **54800.00** | **57540.00** | **3127.78** |
| | **1.5** | **60600.00** | **50800.00** | **45400.00** | **55500.00** | **53500.00** | **53160.00** | **5628.77** |
| | **2** | **56700.00** | **40700.00** | **40000.00** | **40000.00** | **49400.00** | **45360.00** | **7484.85** |
| | **4** | **28500.00** | **29200.00** | **21200.00** | **25600.00** | **15000.00** | **25900.00** | **3187.48** |
| | **8** | **11400.00** | **8860.00** | **9140.00** | **6960.00** | **8140.00** | **8900.00** | **1631.75** |
| | **12** | **3490.00** | **2900.00** | **1720.00** | **1550.00** | **2090.00** | **2350.00** | **822.59** |
| | **24** | **104.00** | **388.00** | **359.00** | **100.00** | **108.00** | **251.80** | **138.28** |
| **G3 (100mg/kg)** | **0** | **0.00** | **0.00** | **0.00** | **0.00** | **0.00** | **0.00** | **0.00** |
| | **0.25** | **94300.00** | **55000.00** | **2100.00** | **134000.00** | **132000.00** | **83480.00** | **55206.25** |
| | **0.5** | **79800.00** | **46700.00** | **15400.00** | **130000.00** | **113000.00** | **76980.00** | **46972.03** |
| | **1** | **96300.00** | **47700.00** | **89300.00** | **93600.00** | **102000.00** | **85780.00** | **21779.51** |
| | **1.5** | **77900.00** | **56100.00** | **84300.00** | **73000.00** | **83900.00** | **75040.00** | **11568.41** |
| | **1** | **90900.00** | **47600.00** | **73600.00** | **74200.00** | **81700.00** | **73600.00** | **16134.28** |
| | **4** | **41300.00** | **36500.00** | **43800.00** | **33900.00** | **53000.00** | **43300.00** | **7920.54** |
| | **8** | **13300.00** | **17200.00** | **16200.00** | **14900.00** | **23900.00** | **17080.00** | **4086.20** |
| | **12** | **3730.00** | **5870.00** | **4810.00** | **4250.00** | **8240.00** | **5380.00** | **1784.94** |
| | **24** | **272.00** | **496.00** | **419.00** | **297.00** | **731.00** | **143. 00** | **184.99** |
| **G4 (200mg/kg)** | **0** | **0.00** | **0.00** | **0.00** | **0.00** | **0.00** | **0.00** | **0.00** |
| | **0.25** | **192000.00** | **165000.00** | **144000.00** | **234000.00** | **60800.00** | **159160.00** | **64455.63** |
| | **0 5** | **243000.00** | **213000.00** | **225000.00** | **214000.00** | **252000.00** | **229400.00** | **17472.84** |
| | **1** | **201000.00** | **183000.00** | **182000.00** | **177000.00** | **227000.00** | **194000.00** | **20566.96** |
| | **1.5** | **198000.00** | **175000.00** | **150000.00** | **173000.00** | **201000.00** | **179400.00** | **20839.87** |
| | **2** | **192000.00** | **166000.00** | **111000.00** | **153000.00** | **173000.00** | **159000.00** | **30306.76** |
| | **4** | **124000.00** | **113000.00** | **57800.00** | **132000.00** | **96800.00** | **164720.00** | **29362.77** |
| | **8** | **77900.00** | **41800.00** | **14200.00** | **45400.00** | **30400.00** | **41940.00** | **23493.57** |
| | **12** | **31900.00** | **1.2900.00** | **3170,00** | **12200.00** | **7970.00** | **13628.00** | **10929.52** |
| | **24** | **3750.00** | **998.00** | **324.00** | **705.00** | **374.00** | **1230.20** | **1434.70** |
| | | | | | | | | |

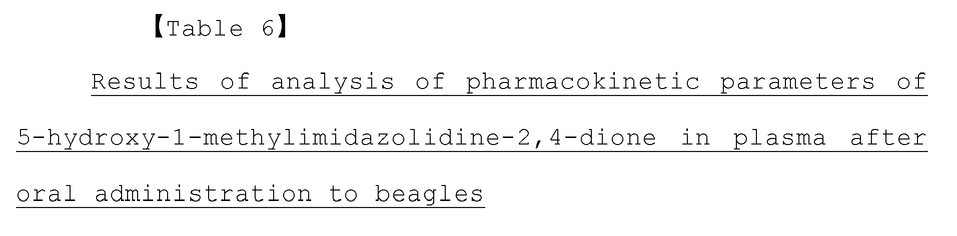

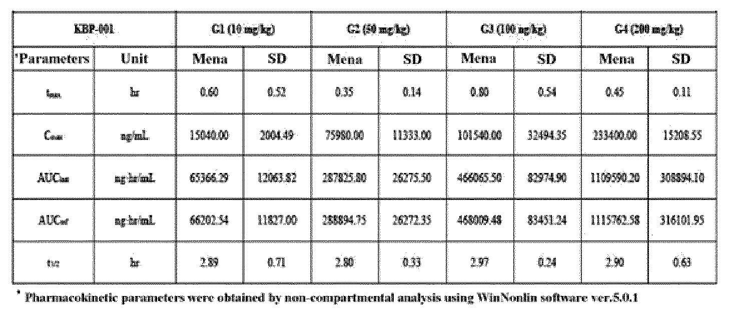

In addition, as can be seen from Table 6, the result of pharmacokinetic parameter analysis showed that, when the compound was orally administered at a dose of 10 mg/kg, the average blood half-life of the compound was 2.89 hours, and AUClₐₛₜ and AUC_{inf} were 65,366.29 (± 12,063.82) ng·hr/ml and 66,202.54 (±11,827.00) ng·hr/ml, respectively.

In addition, it can be seen that, when the compound was orally administered at a dose of 50 mg/kg, the average blood half-life of the compound was 2.80 hours, and AUClₐₛₜ and AUC_{inf} were 287,825.80 ng·hr/ml and 288,894.75 ng·hr/ml, respectively.

In addition, it can be seen that, when the compound was administered orally at a dose of 100 mg/kg, the average blood half-life of the compound was 2.97 hours, and AUClₐₛₜ and AUC_{inf} were 466,065.50 ng·hr/ml and 468,009.48 ng·hr/ml, respectively.

In addition, it can be seen that, when the compound was orally administered at a dose of 200 mg/kg, the average blood half-life of the compound was 2.90 (±0.63) hours, and AUClₐₛₜ and AUC_{inf} were 1,109,590.20 (±308,894.10) ng·hr/ml, and 1,115,762.58 (± 316,101.95) ng·hr/ml, respectively.

### <Example 3>

### Toxicity analysis of 5-hydroxy-1-methylimidazolidine-2,4-dione in beagles

Furthermore, the present inventors performed a toxicity test on 5-hydroxy-1-methylimidazolidine-2,4-dione in beagles. Toxicity tests were conducted based on single oral administration and repeated oral administration for 13 weeks. The stability of the compound was analyzed by evaluating the analysis items including clinical symptoms, weight, feed intake, blood test, urine and eye test during the administration period.

5-Hydroxy-1-methylimidazolidine-2,4-dione was administered at doses of 500, 1,000, and 2,000 mg/kg once orally to beagles, the following items were observed for 2 weeks, and toxicity analysis upon repeated administration was performed in the following method after repeated oral administration once a day for 13 weeks.

Meanwhile, the following items were observed for two weeks after single oral administration. The result showed that no special abnormal symptoms were found in any of the items. This means that the compound of the present invention does not cause toxicity in beagles within the treatment dose range.

Furthermore, the following examples show the experimental methods of toxicity analysis during repeated oral administration for 13 weeks and the results thereof.

### Observation of general symptoms

General symptoms (such as vomiting, salivation, diarrhea, and abnormal behavior) and death were observed daily during the 13-week test period, starting from the day before administration, and abnormalities were recorded in clinical symptoms.

### Weight measurement

During the 13-week test period starting from the day before administration, the weight was measured a total of 15 times before administration (pre), before administration on the day of administration, and every week over the 13-week test period after the first administration at a predetermined time (remaining feed measurement time) before administration of the test substance (on every Tuesday during the test period).

### Feed intake

During the 13-week test period starting from the day before administration, feed intake was measured a total of 14 times before administration (pre) and every week on the weight measurement day for 13 weeks after the first administration. Approximately 200 g/day/head of solid feed was injected into a feed container at a predetermined time on the day before the weight measurement day and consumed freely. After 24 hours, the remaining feed was measured the next day.

### Fasting

Fasting was conducted the day before administration (blood test date, pre), the 4^{th} week (day 29) after administration, the 8^{th} week (day 57) after administration, and the 13^{th} week (day 92) after administration, and when the remaining feed was present in the feed container 3 hours after feeding on the day of fasting, it was removed.

### Hematological test

All subjects were fasted for 16 hours and measurements were then performed pre (before administration), 4 weeks (day 29) after administration, 8 weeks (day 57) after administration, and 13 weeks (day 92) after administration. Within 3 hours after 2 to 3 ml of blood collected from the jugular vein of each subject was injected into a CBC bottle containing EDTA, RBC, HGB, HCT, RBC indices (MCV, MCH, MCHC), and platelet count and WBC differential count were measured using an automatic hematometer.

### Blood biochemistry test

5 ml of collected blood was placed in a serum separation tube (SST) and centrifuged at 4°C at 4,000 rpm for 10 minutes, and then serum was collected therefrom and used for biochemical analysis. Blood urea nitrogen (BUN), albumin (ALB), creatinine (CREA), total bilirubin (T-BIL), direct bilirubin (D-BIL), glucose (GLU), triglyceride (TG), total cholesterol (CHO), AST (GOT, Glutamate Oxaloacetate Transaminase), ALT (GPT, Glutamate Pyruvate Transaminase), alkaline phosphatase (ALP), lactate (LAC), creatinine kinase (CPK), lactate dehydrogenase (LDH), calcium (CA), phosphorus (PHOS), and total protein (TP) (total 17 items) were measured using a blood biochemistry analyzer (AU480 Chemistry Analyzer BECKMAN COULTER inc.)

### Blood coagulation test (PT, aPTT, Fibrinogen)

2 ml of collected blood was placed in a 3.2% sodium citrate tube, and centrifuged at 4,000 rpm for 10 minutes at 4°C, and plasma was collected for blood coagulation analysis. Prothrombin time (PT), activated partial thromboplastin time (aPTT), and fibrinogen (FIB) were analyzed using a blood coagulation measuring device (Werfen ACL ELITE Pro).

### Electrolyte test

Na+, Cl-, and K+ in 0.5 ml of plasma obtained in the blood biochemical test were assayed using an electrolyte measuring device (i-Smart^{®} 30 Electrolyte Analyzer, Korea).

### Urine collection and urine test

Urine tests were performed on the urine collected in the morning of blood donation day (Wednesday of the week) after fasting for about 16 hours, before administration, 4^{th} week (day 29) after administration, the 8^{th} week (day 57) after administration, and 13^{th} week (day 92) after administration. Urine was collected from all the fasting subjects using a silicone tube connected to a duct after mounting a urine plate at the bottom of the cage for 16 hours. The collected urine was centrifuged at 1,500 rpm for 5 minutes to obtain the supernatant, then immersed in a urine strip (thinka Urine Test Strip, ARKRAY, Inc., Japan), the residue around the strip was removed, urine was analyzed using a urine analyzer (thinka RT-4010, ARKRAY, Inc., Japan), and the urine specific gravity of each subject was measured using a urine hydrometer (Master-URC.NM, ATAGO CO., LTD, Japan).

### Eye Test

Eye test were conducted in the afternoon in the blood test day and blood donation day before administration (pre), the 4^{th} week (day 29) after administration, the 8^{th} week (day 57), and the 13^{th} week (day 92) after administration. After observing the appearance of the left and right eyes on all the subjects, both the left and right eyes were dilated with a mydriatic agent (Mydrin^{®}-P eye drops, ingredients: tropicamide/phenylephrine hydrochloride Taejun Pharmaceutical), and then the vitreous body and retina were tested with an ophthalmoscope (Vantage Plus LED Convertible Wired, Keeler Instruments Inc. USA).

Weight was measured a total of 15 times for each group during the test period, and was maintained in all groups compared to before administration, or increased in some subjects. No sharp decrease in weight was observed during the test period in both male and female subjects, no significant differences in weight from the control group was observed, no significant difference in feed intake between respective groups was identified, and no feed residue was observed in all subjects (Table 7). In addition, no unusual symptoms (vomiting, salivation, diarrhea, behavioral abnormalities, etc.) or deaths occurred during the test period.

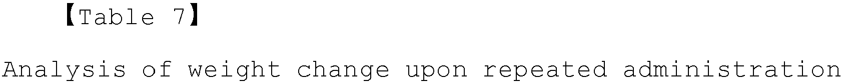

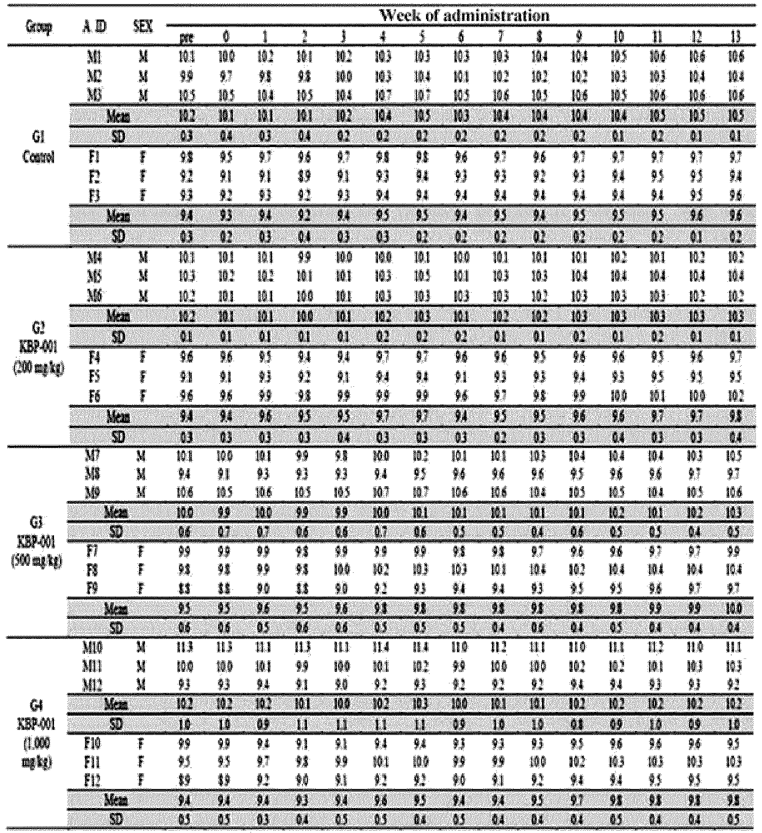

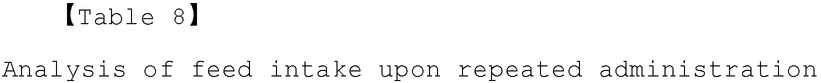

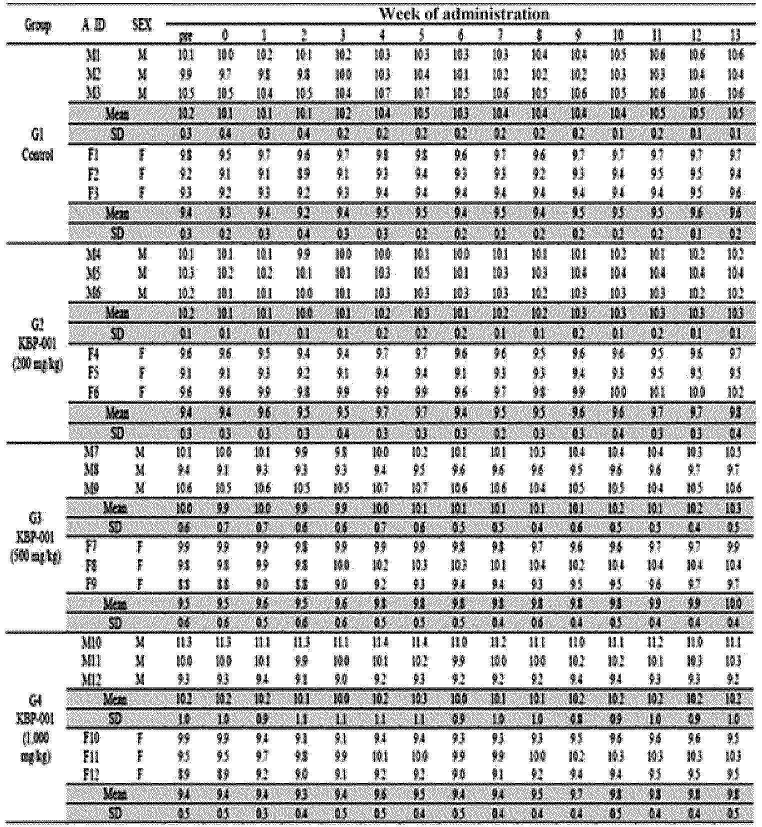

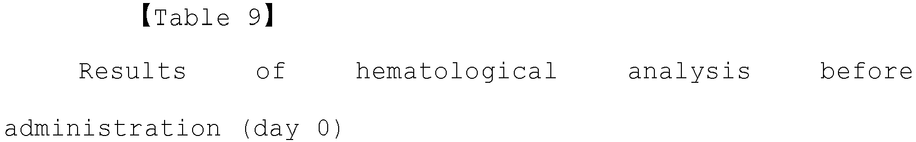

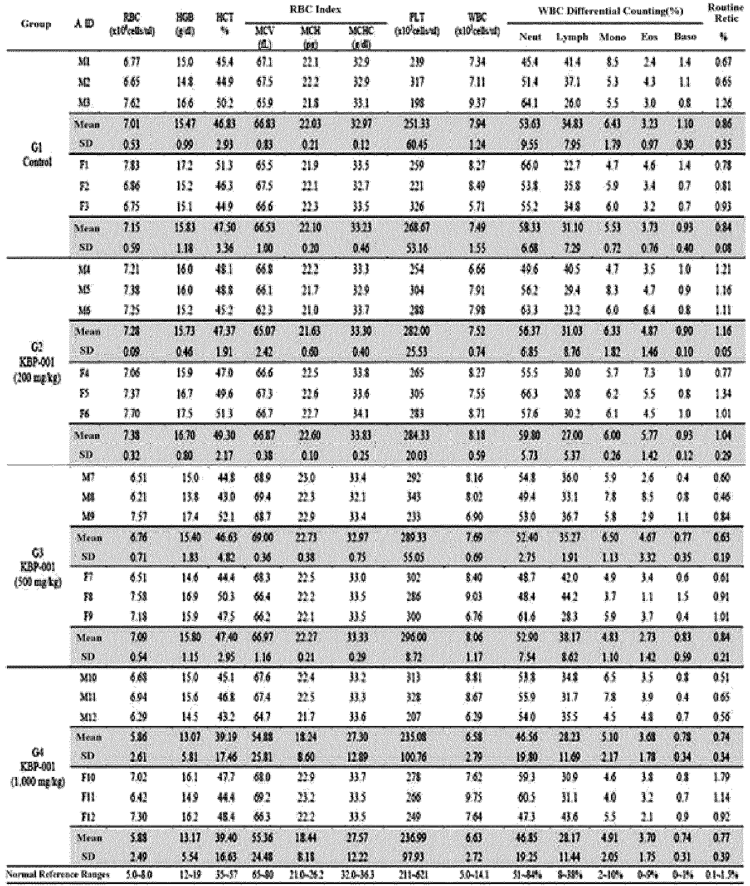

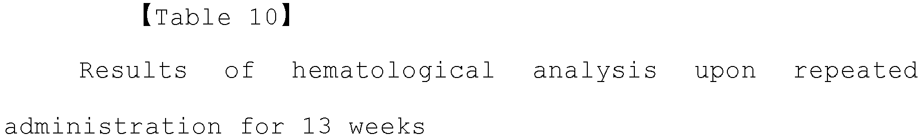

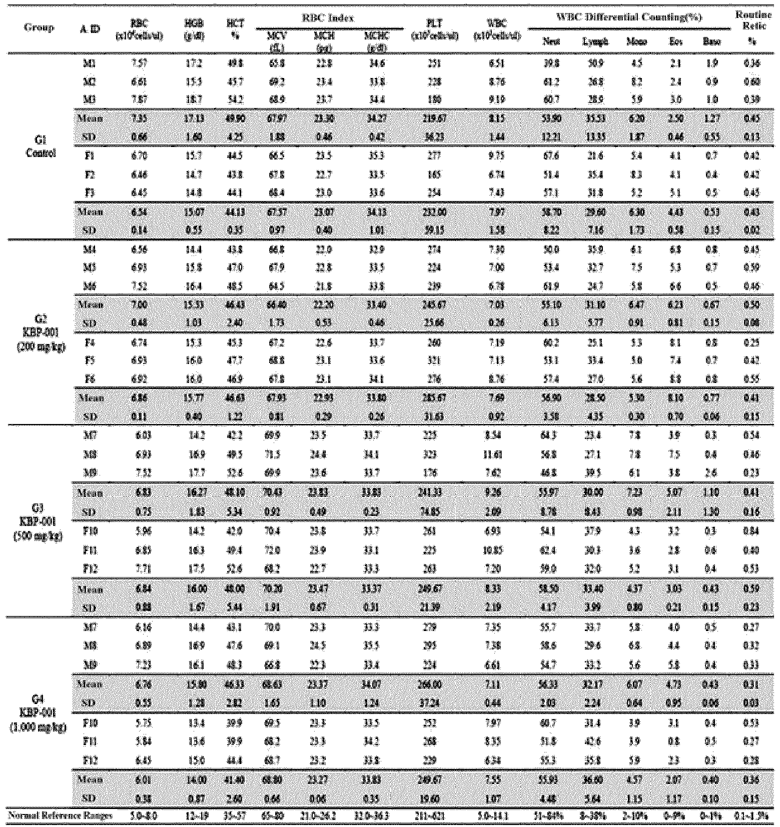

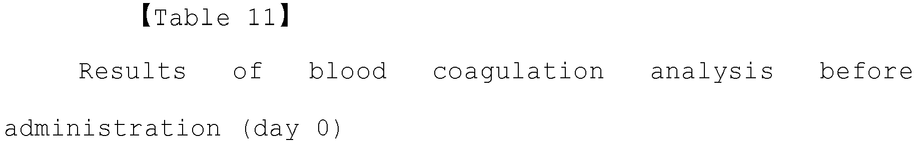

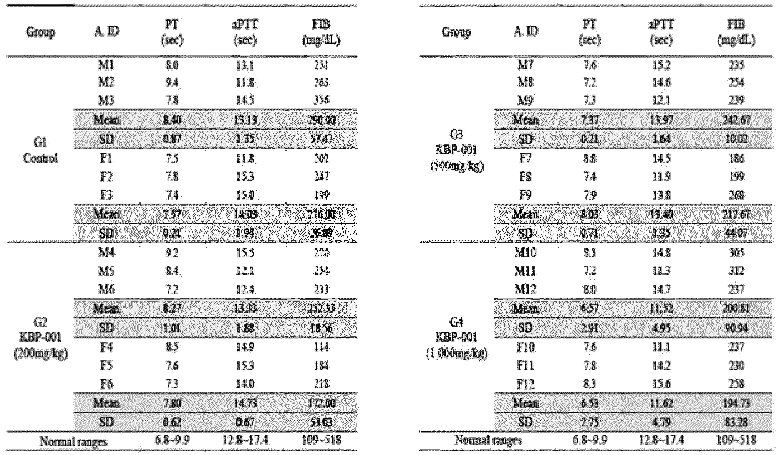

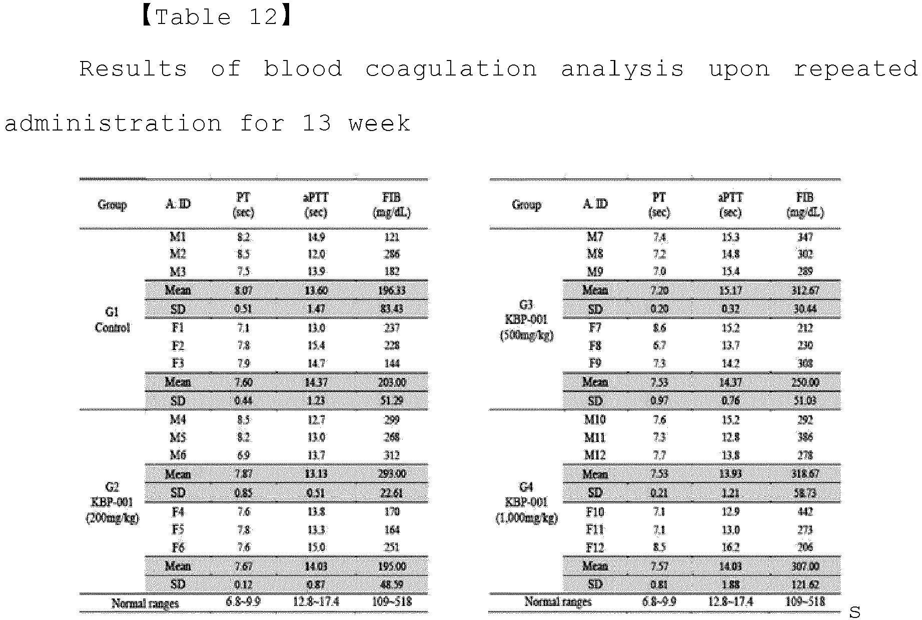

In addition, the results of blood biochemistry and electrolyte test showed that, overall, all test indicators including liver levels (GOT, GPT, ALP) and kidney levels (BUN, ALB), and Na+, K-, and Cl- test items in all test groups fell within the normal physiological ranges before and after administration and no changes due to the effects of compound (drug) treatment compared to the control group were observed (Tables 13 to 16).

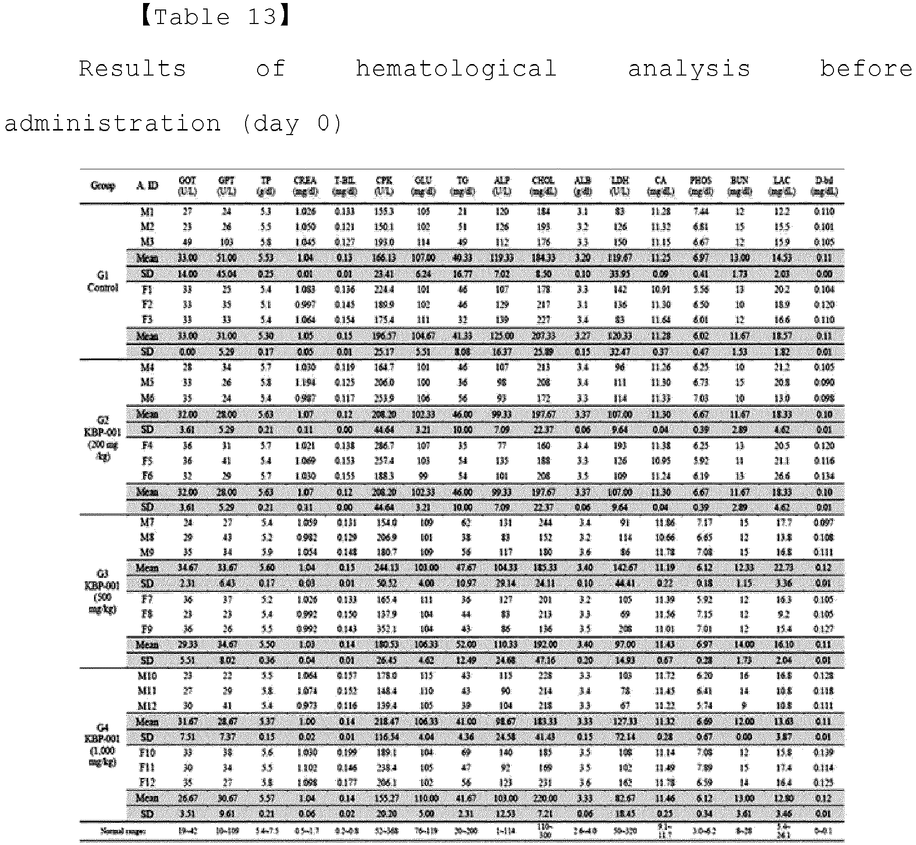

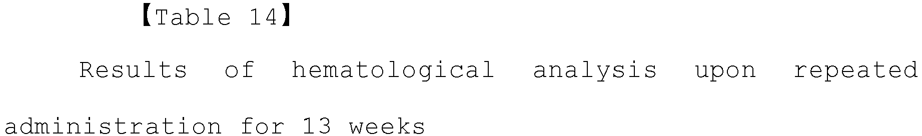

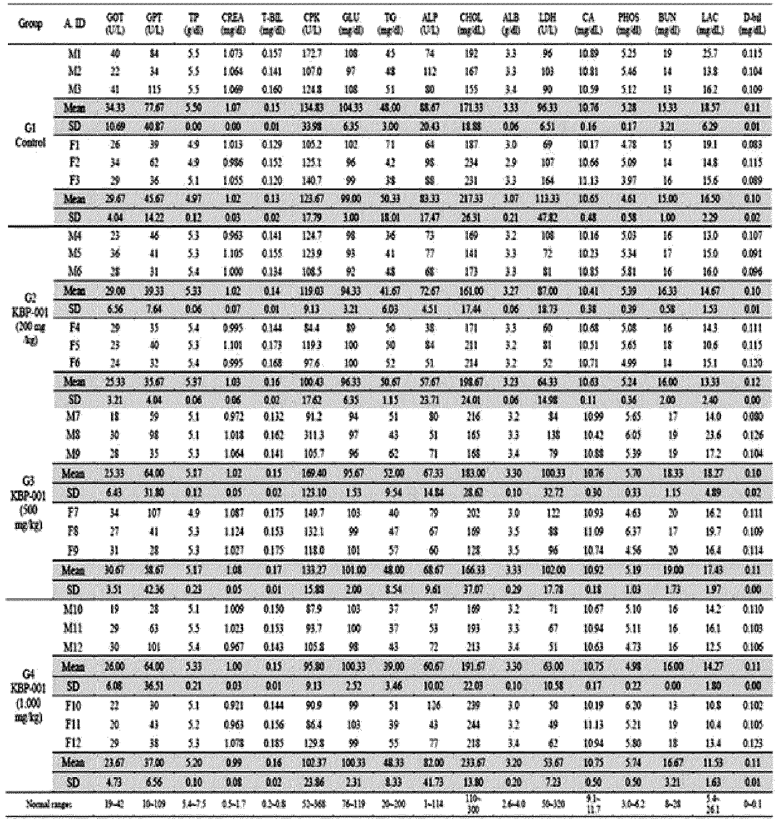

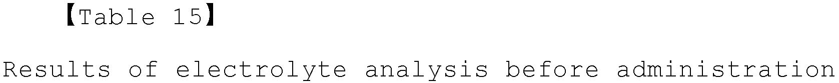

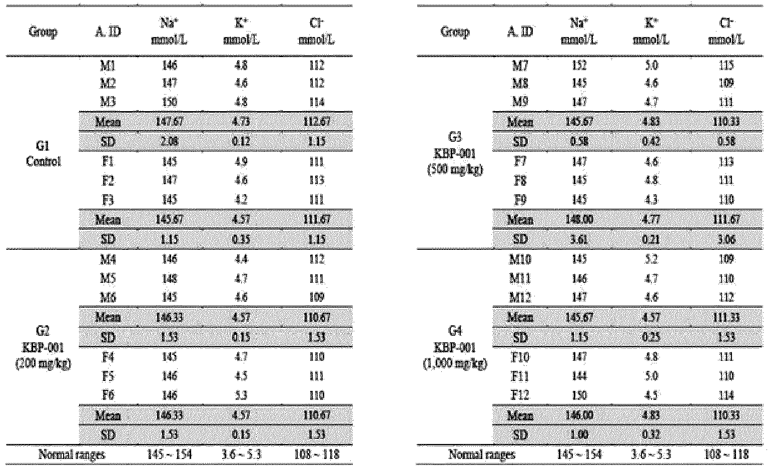

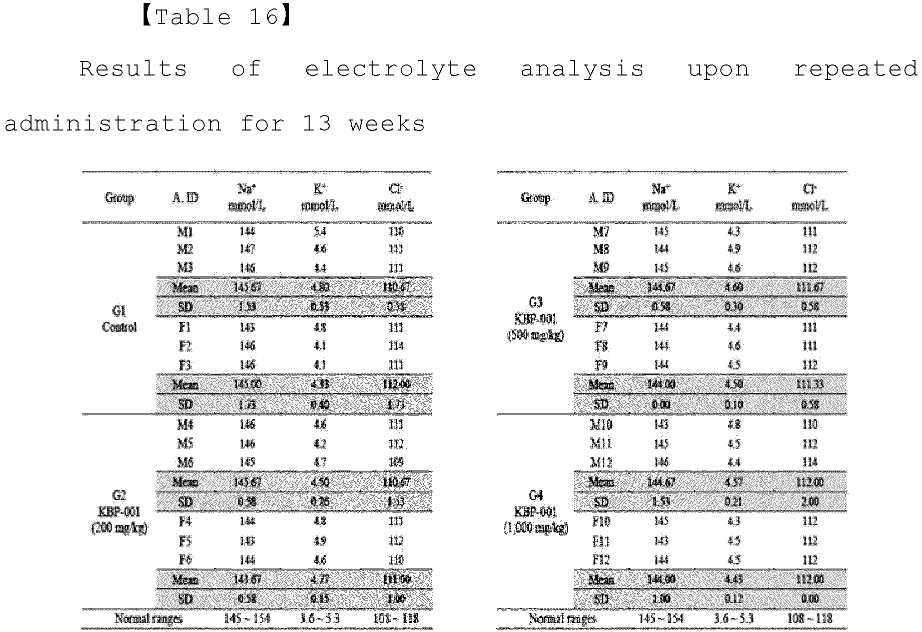

In addition, the result of the urine test showed that the numerical results of all test items including urine specific gravity before and after administration of the compound fell within the normal physiological ranges and no changes were observed due to administration of the compound of the present invention. The result of eye test showed that no specific findings were observed before and after compound administration (Tables 17 to 18).

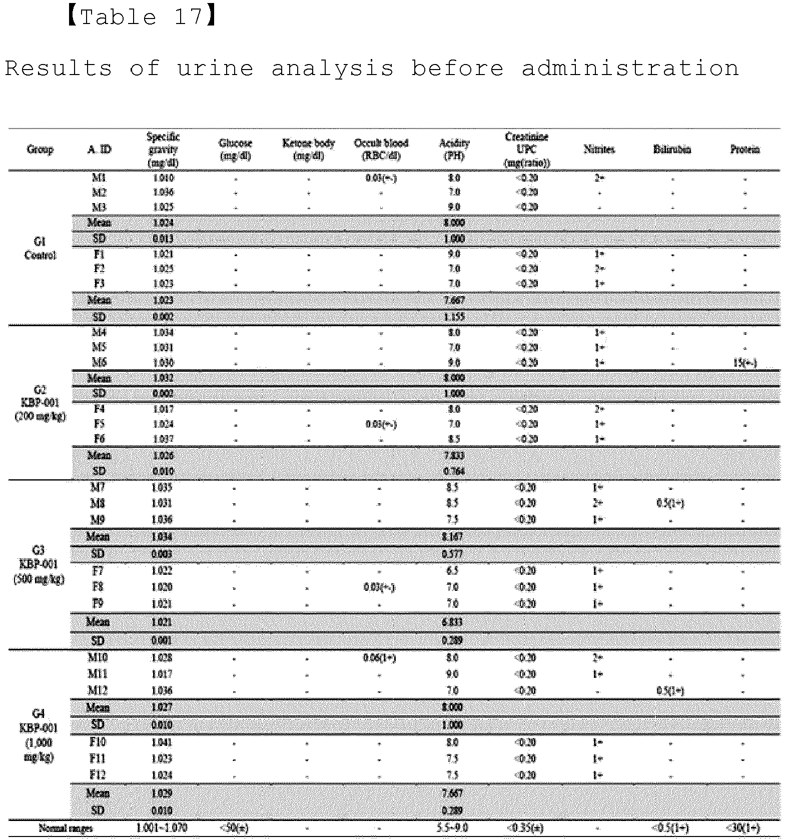

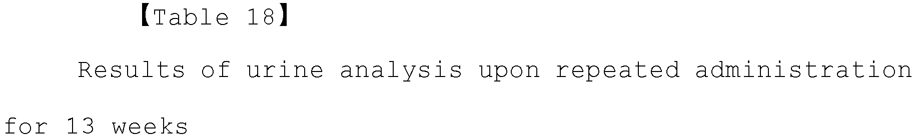

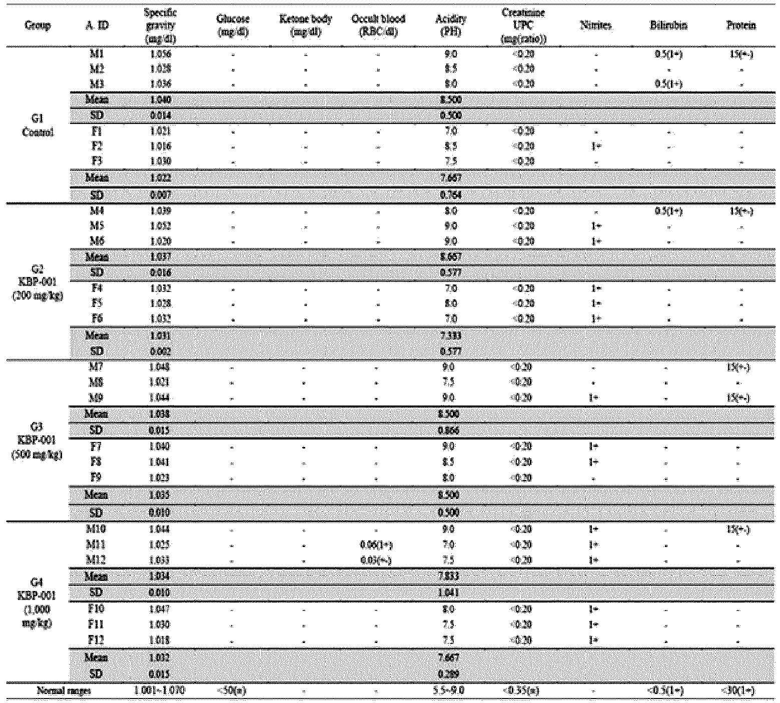

These results showed the present inventors determined that the 5-hydroxy-1-methylimidazolidine-2,4-dione does not cause toxicity or side effects in animals, and is useful as a veterinary medicine that can effectively prevent or treat kidney disease in companion animals (dogs and cats) .

Although the present invention has been be described in more detail with reference to specific embodiments, the embodiments are provided only for illustration and thus should not be construed as limiting the scope of the present invention. Those skilled in the art will appreciate that various modifications, additions and substitutions are possible, without departing from the scope and spirit of the invention as disclosed in the accompanying claims. For example, each component specifically disclosed in the embodiments may be modified. In addition, these differences relating to modifications and applications should be construed as falling within the scope of the present invention as defined in the appended claims.

## Claims

1. A composition for preventing or treating kidney disease in animals containing 5-hydroxy-1-methylimidazolidine-2,4-dione or a pharmaceutically acceptable salt thereof as an active ingredient.

2. The composition according to claim 1, wherein the kidney disease is selected from the group consisting of nephritis, pyelonephritis, nephrotic syndrome, kidney cancer, acute pyelonephritis, chronic pyelonephritis, renal tuberculosis, urinary tract infection, urinary stone (calculi), ureter stone, acute renal failure, chronic renal failure, diabetic nephropathy, chronic glomerulonephritis, acute progressive nephritis, nephrosis syndrome, microglial glomerulosclerosis, membranous nephropathy, and membranous proliferative glomerulonephritis occurring in animals.

3. The composition according to claim 1, wherein the animal is a dog or a cat.

4. The composition according to claim 1, wherein the 5-hydroxy-1-methylimidazolidine-2,4-dione is orally administered at a daily dose of 25 to 200 mg per kg of weight to an animal with chronic kidney disease having a serum creatinine (sCr) level of 1.4 mg/dL or higher.

5. The composition according to claim 4, wherein the dose is 25 mg to 100 mg per kg of weight.

6. A method of treating kidney disease in animals comprising administering a composition containing 5-hydroxy-1-methylimidazolidine-2,4-dione or a pharmaceutically acceptable salt thereof to an animal with kidney disease.

7. The method according to claim 6, wherein the animal with kidney disease is an animal with chronic kidney disease having a serum creatinine (sCr) level of 1.4 mg/dL or higher.

8. The method according to claim 6, wherein the kidney disease is selected from the group consisting of nephritis, pyelonephritis, nephrotic syndrome, kidney cancer, acute pyelonephritis, chronic pyelonephritis, renal tuberculosis, urinary tract infection, urinary stone (calculi), ureter stone, acute renal failure, chronic renal failure, diabetic nephropathy, chronic glomerulonephritis, acute progressive nephritis, nephrosis syndrome, microglial glomerulosclerosis, membranous nephropathy, and membranous proliferative glomerulonephritis occurring in animals.

9. The method according to claim 6, wherein the animal is a dog or a cat.

10. The method according to claim 6, wherein the administration is oral administration.

11. A functional feed for preventing or ameliorating kidney disease in animals containing 5-hydroxy-1-methylimidazolidine-2,4-dione or a pharmaceutically acceptable salt thereof as an active ingredient.
